(19) Europäisches Patentamt
European Patent Office
Office européen des brevets

(11) EP 2 832 285 A1

(12) **EUROPEAN PATENT APPLICATION**

(43) Date of publication:
04.02.2015 Bulletin 2015/06

(51) Int Cl.:
*A61B 5/00* (2006.01)     *A61B 17/16* (2006.01)

(21) Application number: 13003805.2

(22) Date of filing: 31.07.2013

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(71) Applicant: ETH Zurich
8092 Zurich (CH)

(72) Inventors:
• Schellenberg, Florian
CH - 8331 Auslikon (CH)
• Lorenzetti, Silvio
CH - 8260 Stein am Rhein (CH)

(54) **Surgical machining instrument and method for determining the local mechanical resistance of a bone**

(57)     A surgical machining instrument (10), especially a surgical drilling or sawing instrument, is used for determining the local mechanical resistance of a bone (50) in connection with the machining action. Therefore, the instrument comprises a first sensor unit adapted to determine energy expenditure during the machining action and a second sensor unit (30, 40) adapted to determine the volume of removed material during the machining action and being time synchronized with the first sensor unit. A control unit (20) is adapted to determine the applied specific energy as energy per volume of removed material over time based on the data of the sensor units and is furthermore adapted to convert the measured data in a value of the local mechanical resistance of said bone.

Fig. 1

EP 2 832 285 A1

**Description**

TECHNICAL FIELD

The present invention relates to a surgical machining instrument, especially to a surgical drilling or sawing instrument and relates to a method for determining the local mechanical resistance of a bone. It further relates to a system and method comprising such a surgical machining instrument and an x-ray apparatus for determining the local mechanical resistance of a bone.

PRIOR ART

Drilling and sawing are two important machining methods within any surgical intervention wherein the surgeon prepares the setting of e.g. implants, nails or artificial joints. Therefore it is important for the result of the intervention to know about the quality of the bone material within which the holes are drilled or where parts are cut away to allow setting of implants. Such techniques of drilling and sawing are generally addressed under the DIN norm 8589. Machining as defined here relates to such activities.

DE 10 2009 033 364 A1 relates to a measuring device for intra-operative determination of bone quality of bone portion. It has a measuring unit, which is connected with a rod-shaped measuring sensor. Said device allows determining the strength of the bone within an existing drill hole through application of a drill probe to be entered in the existing hole.

US 6,591,698 provides a surgical instrument for screwing in a surgical implant or for cutting a thread in human bone tissue. This instrument comprises a control unit supplying power and controlling the speed of an electric motor. Detection members transmit signals dependent on the torque of the instrument for display on a screen for an assessment of the quality of the implant or of the bone tissue.

WO 00115138 A1 discloses an instrument with a drill portion to drill dentin. The instrument comprises an electric motor of which the torque can be sensed. A torque against time curve is provided to assess bone quality.

A mechanical device for measuring the local mechanical resistance of a porous body as e.g. bone material is disclosed in US 2008/0300510 A1 by inserting a suitable tool into a previously drilled hole.

A surgical machining instrument with the features of the preamble of claim 1 is disclosed in US 2011/245833, showing a drill including a sensor unit and a control unit with software capable of being programmed to continuously measure and/or control a variety of functions including bone depth, material strength, bone density, skive, drill bit development, speed of rotation, acceleration, deceleration, irrigation, voltage, torque, thrust, feed rate, current, voltage, axial movement, axial force and other functions of the instrument. The aim of the measurements by the sensor unit of this prior art device is to detect specific situations of the drilling actions, as to enable the control unit to provide information to the user pertaining to the passage of the tool through varied layers of tissue, e.g. through cortical bone into medullary canal or cancellous bone, medullary canal or cancellous bone to cortical bone, or from cortical bone to soft tissue, i.e. measured data by the sensor unit is transformed in an information relating to the local material in the sense that the person using the drill knows where the tip of the drill bit is in.

US 2011/245833 mentions to use dual energy x-ray absorptionsmetry, in short DEXA, to measure bone density off-line and to use such measurements for osteoporosis treatments. The document also states that this x-ray absorption measurement is necessarily an offline measurement.

DEXA or DXA is a means of measuring bone mineral density (BMD). Two x-ray beams with different energy levels are aimed at the patient's bones. When soft tissue absorption is subtracted out, the BMD can be determined from the absorption of each beam by bone. Average bone mineral density is defined to be BMC / W [g/cm2] whereas BMC is the bone mineral content in g/cm and W is the width at the scanned line in cm.

SUMMARY OF THE INVENTION

Based on this prior art it is an object of the invention to give online a more reliable assessment of the bone quality into which the instrument is machining, e.g. drilling a hole, cutting a thread or cutting a part. Obtaining such information is a valuable object of the invention, since surgeons have to decide - during an intervention - about the choice of specific screws to be used in osteoporotic or healthy bone material, i.e. depending on the local material properties and the mechanical resistance of the bone. This also relates to the choice of use or kind of bone cement or the choice to provide additional fixation elements.

It is further an aim of the present invention to provide a method for an, especially ex situ, evaluation of the local properties of a material, especially bone material. This evaluation can be conducted independent of a specific surgical intervention on a person and can therefore relate to a non-surgical intervention.

This and further objects of the invention are achieved by an instrument having the features of claim 1.

A surgical machining instrument, especially a surgical drilling or sawing instrument, for determining the local material properties, especially the mechanical resistance of a bone, in connection with the machining action, comprises a first sensor unit adapted to determine energy expenditure during the machining action and a control unit adapted to convert the measured data in a value of the local material properties. The instrument also comprises a second sensor unit adapted to determine the volume of removed material during the machining action and being time synchronized with the first sensor unit. Now the control unit is adapted to determine the applied specific energy as energy per removed material, especially bone, volume over time based on the data of the sensor units and to convert the measured data in a value for the mechanical resistance of said bone.

During the machining action, the specific energy as energy per volume is measured for the determination of the bone quality. In order to obtain the necessary measuring information, different approaches are used according to the machining device; in case of an electric drill, the energy is calculated based on the current consumption and voltage as measured over time. Furthermore the removed bone volume is also determined over time. Based on this data the specific energy is calculated dependent on the drilling tool or saw which determines a key figure or characteristic factor for the bone quality. This value is determined in real-time through time synchronized measurements of removed volume and energy applied to the instrument for the removal action.

The control unit is adapted to take into account as parameter the kind of machining tool, especially if it is a drilling tool or a saw, especially the diameter of the drill or the thickness of the saw blade, respectively.

The second sensor unit can comprise a depth gauge adapted to monitor, during the machining, the advance of the machining instrument into the material to determine the volume of removed material. In a different approach the second sensor unit comprises an x-ray apparatus and an x-ray detector adapted to monitor, during the machining, the advance of the machining instrument into the material to determine the volume of removed material.

When the machining instrument is electric driven, the first sensor unit can comprise sensors detecting current consumption and applied voltage over time. When the machining instrument is air driven, then the first sensor unit can comprise sensors detecting the mass flow or the air velocities of such an air driven instrument.

In a specific embodiment said specific energy as energy per volume is calculated on measured values of current consumption and applied voltage over time of an electric driven machining tool.

Of course said values are calibrated against the idle consumption. In principle only the time of drilling or sawing is taken into account. These two options, calibration and time measurement, can also apply for an air driven instrument.

The control unit is further adapted to take into account as parameter the kind of machining tool, especially if it is a drilling tool or a saw. This also comprise known values of drill diameters, kind of drill etc.. As mentioned the machining instrument can be a drill and the control unit is adapted to take into account as parameter the diameter of the drill and/or the length of the drilled hole. It is also possible that the machining device is a saw or another device removing bone wherein, during the machining action, the applied specific energy as energy per removed bone volume can be determined.

A system comprising such a surgical machining instrument can also have a dual energy x-ray apparatus (i.e. DEXA) for determining a further value of the local mechanical resistance of a bone in connection with the machining action. Then the control unit is adapted to receive measured dual x-ray beam data from an x-ray detector relating to the local bone portion before machining takes place, i.e. the bone to be machined. In such a case a series of images at different energy values of the x-ray apparatus are taken and then the control unit is further adapted to calculate the calcium content and therefore the calcium based stability of the bone obtained through said dual energy x-ray measurement on known ways and to calculate the collagen based stability of the bone taking into account the overall stability of the bone calculated based on said measured energy consumption per volume data related to the local mechanical resistance of said bone.

A method for determining the local mechanical resistance of a bone being machined in situ or ex situ comprises the steps of determining the energy consumption for the local bone portion being machined by a surgical machining instrument, especially a surgical drilling or sawing instrument. Said values are detected by a first sensor unit. Then the volume of removed material during the machining action is detected by a second sensor unit. These values are used to calculate, during the machining action, the applied specific energy as energy per removed bone volume based on the energy consumption, e.g. current and voltage values when an electric driven instrument is used, varying over time, and convert the measured data in a value of the local mechanical resistance of said bone.

Within such a method the current consumption and the applied voltage is measured over time and integrated in said energy per removed bone volume value. In other words, the removed bone volume and the power based on current consumption and applied voltage is measured and used for the integration steps. This can be done by applying the formula

$$\text{material property} \sim k_1\left(\frac{\int_{t=0}^{T} U(t)I(t)dt - \int_{t=0}^{T} U_0(t)I_0(t)dt}{V(T)}\right)^{k_2} + k_3$$

$k_{1...n}$   constant

$U(t)$   voltage over time

$I(t)$   current over time

$T$   machine action time

$U_0$   base line voltage

$I_0$   base line current

$V(t)$   removed volume

as developed by the applicant.

From Tissue Mechanics, Springer, ISBN: 0-387-36825-6, S. cowin and S. Doty it is known that cancellous bone is not only anisotropic but also very inhomogenous due to significant variation in the solid volume fraction that occurs in any whole bone. The method disclosed here enables the determination of the mechanical resistance of a bone being machined dependent on drilling depth, thus enables to separately determine the mechanical resistance of the cancellous and the cortical bone.

The method can further comprise the step of determining a calcium based stability value for the local bone through dual energy x-ray detection of an x-ray beams transmitted through the bone portion in question before machining, wherein a collagen stability value for the local bone is subsequently calculated through deduction from said overall local mechanical resistance of said bone.

Therefore, on one hand, a depth gauge can be used as a first sensor unit or a possible embodiment uses a so-called C-arm x-ray unit which encompasses an x-ray source and an image intensifier. The C-arm sensor provides real time information relating to the removed bone volume. It can be part of the removed volume sensor unit.

Beside this x-ray device providing data in continuous as a film, it is also possible that the system and method comprises such a surgical machining instrument and beside the first x-ray system to quantify the removed bone material volume a supplementary dual energy x-ray absorptiometry (DEXA) apparatus for determining the local material properties, especially the bone properties, e.g. the BMD and BMC values. In this case it is especially an advantage that the measurement can be done online and without a specific waiting time. The single DEXA images are just taken in advance of the drilling/sawing process.

The surgical instrument according to an embodiment of the invention uses the sensor unit adapted to determine, during the machining action, the applied specific energy as energy per removed material, wherein it determines the energy as a function of time as well as the removed material volume as a function of time from a second time synchronized sensor unit, resulting in the applied specific energy as energy per removed material.

The surgical instrument can then use the control unit adapted to convert the measured data in a value of the local material properties converts said data real-time based on said mathematical correlation.

Furthermore the time synchronized second sensor unit comprises within the surgical instrument at least one sensor detecting the volume of the removed material as a function of time. Then, preferably, the at least one sensor is a pulsed x-ray or a depth sensor taking a constant drill bit diameter into account.

Further embodiments of the invention are laid down in the dependent claims.

BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,

Fig. 1   shows a very schematical representation of the application of the surgical machining instrument to a bone;

Fig. 2   shows a schematical representation of the important parameters to be measured through a control unit for an instrument according to Fig. 1;

Fig. 3   shows a schematical representation of a workflow provided by the control unit of the surgical instrument or the surgical system;

Fig. 4   shows a representation of a power vs. time curve used within the calculation in the control unit; and

Fig. 5   shows a representation of a measured graph of power vs. time curve used within the calculation in the control unit.

DESCRIPTION OF PREFERRED EMBODIMENTS

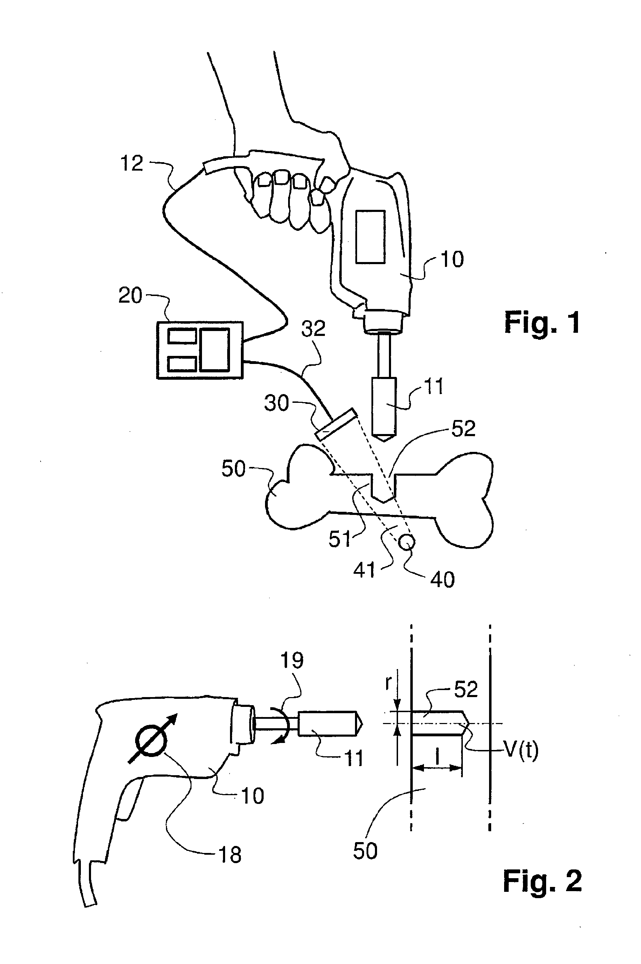

Fig. 1 shows a very schematic representation of the application of the surgical machining instrument 10 to a bone 50. The surgical instrument 10 in the embodiment shown is a drill having a drill bit 11 attached to drill a hole 52 in the bone 50. The surgical instrument 10 is connected to a control unit 20 via a connection 12 which allows measuring the power consumption and further parameters of the drill during the drilling action.

The drill can be an electric driven or an air driven machining instrument.

In an advantageous embodiment the local region 51 of the bone where the drilled hole 52 is provided is monitored by an x-ray source 40 providing an x-ray beam 41 which is detected by the x-ray detector 30. The signals detected are provided via connection 32 to the control unit 20 for further perusal as explained below.

In a different embodiment the x-ray source/sensor assembly can be replaced by a mechanical or optical depth gauge device as will be explained below.

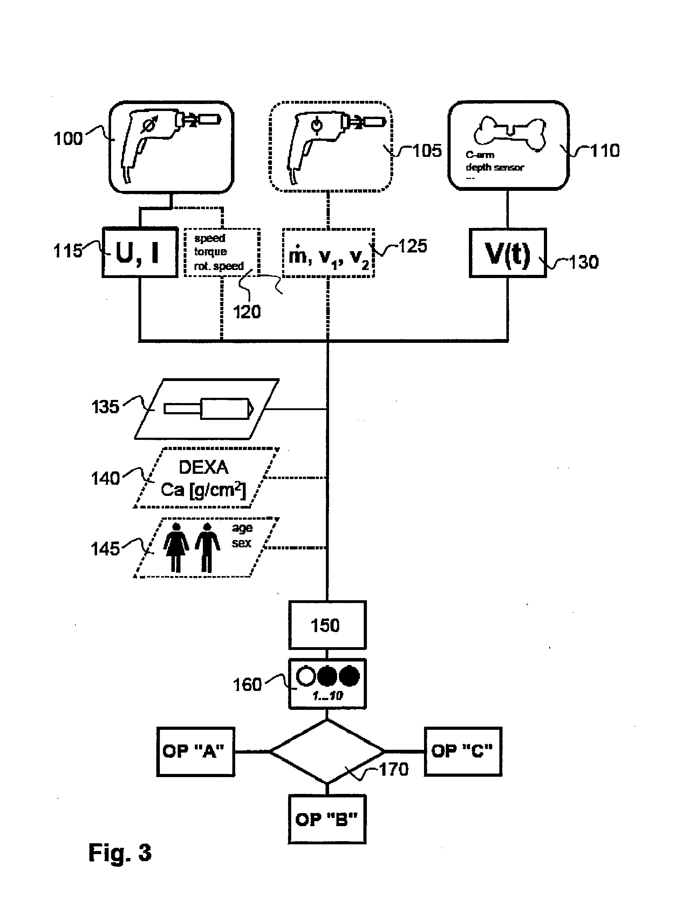

Fig. 2 shows a schematical representation of the important parameters to be measured through the control unit 20. The electric driven drill 10 has during its actuation a current consumption 18. Within an air driven embodiment similar values can be derived from mass flow or air velocity of such an air driven instrument. Drilling is connected with a rotation of the drill bit which is measured as revolutions per time unit 19. Furthermore, the torque connected with this drill action can be measured. The drill bit 11 itself has specific properties which are known in advance and as such predetermined. Such properties may include diameter, kind of the drill helix; material of the drill etc.. The drilled hole in bone 50 has a specific length or depth I and is connected with a radial diameter r. These parameters together with further power parameters at the voltage used within the drill are provided in a workflow which is shown in Fig. 3 provided by the control unit 20 of the surgical instrument 10 or the surgical system with additional x-ray analysis. If the drill hole is not a blind hole, the length of the drilling action can be determined e.g. by such x-ray analysis. It is also contemplated that a mechanical depth gauge is used to evaluate the position of the tip of the drill bit 11 and the removed material in the hole 52.

The parameter measured through the electric driven drill as system 100 are especially voltage U and current I, shown in box 115. Additionally and therefore marked in dotted lines in box 120, can be added the speed as revolution per minutes, a measured torque applied to the bone during the drilling action and the speed of the drill in the longitudinal direction of the piercing (i.e. the advance against time).

Additional parameters to be added are the specification of the drill bit, the length of the hole drilled 110 and further parameters. These may include the result of an x-ray measurement (mentioned as C-arm in box 110, when x-ray source and detector encompass the bone to be evaluated in the shape of a "C"). It is an advantage to combine the calculation of the energy applied to the bone from the power consumption (electric or air driven etc....) with a specific analysis result of the x-ray image. It is possible to determine the calcium content in the bone separately through the x-ray measurement and the result of the independent power measurement for the strength of the bone can therefore be improved since collagen based stability of the bone is not detectable via the simple x-ray measurement. This is explained in box 140, which can be especially a DEXA scan. In fact, the combination of two x-ray absorption measurements of different energy levels in real time allows to obtain separate collagen based stability from the calcium based stability for the specific bone monitored. The calcium based stability is obtained through x-ray measurements and the collagen based stability is then calculated from the measured combined stability (through power determination of the DEXA scan) deducing the calcium based stability allowing a more complex result of the measurement and the drill properties, e.g. drill bit parameters 135.

Furthermore, user specific data as possible data related to the owner of the bone can be added under box 145, as age, sex etc.. Then these parameters are taken together and calculated in box 150 to give an indication relating to the bone quality which is shown as a screen lamp 160. This can be a traffic light like indication as red, yellow and green for the quality of the bone material or an abstract figure from e.g. 1 to 10. This result can be fed into a comparison device 170 which is adapted to provide adapted physical parameters for surgical interventions as specific implants or screws to be used in connection with such a drill hole 50 inside the measured bone.

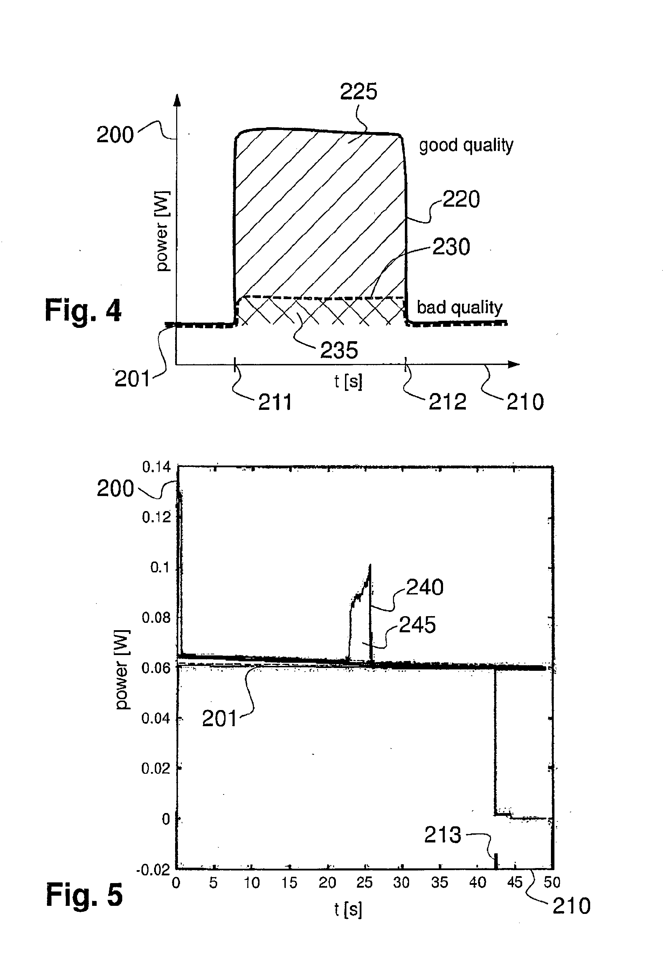

Fig. 4 shows a representation of a power versus time curve used within the calculation 150 in the control unit 20. Power 200 is shown against time 210. Initially at the ideal state there is a power consumption 201 of the device at the point in time 211 the drilling takes place and ends at the second point in time 212. For a good quality bone usually the power consumption 220 is high and therefore the hatched surface 225 between the power value 200 and the ideal state 201 is the total power consumption used by the drill bit to drill the entire hole. Curve 230 shows a further drill event, drafted as to happen between the same time point 211 and 212. In this example, the surgeon would apply the same advance of piercing speed in both cases. However, the procedure is independent of the piercing speed. Since the bone quality of this second curve 230 shown as a dotted line is far less good, the power consumption during the drilling is smaller and the hatched integral of the power over time, reference number 235, is smaller.

Fig. 5 shows a measured representation of a graph of power 200 against time 210 with the ideal rate 201 at the value 0.06 for the power and wherein the motor is stopped at the point in time 213 dropping the power consumption to 0. Here the measured drill curve 240 has a plateau face with increasing power levels until the end of the drilling action and reference number 245 represents the integral of the power over time.

The drilling action shown in Fig. 5 used a drill bit 11 with a diameter of 6 mm. The bore hole depth was 25,9 mm which calculated to a bore hole volume of 733 mm$^3$. The energy used was 0.68 Joule. With a specific energy of $9.35 \times 10^{-4}$ Joule x mm$^{-3}$, the bone compression strength was 2.2 MPa. Since the bone used was a specific artificial bone, and thus tested ex situ, the results could be compared with the expected values and the measurement results were in accordance with the estimated bone strength of this artificial bone.

Of course different drill diameters can be used, especially between 1 and 12 mm. The bore hole depth which is usually applied lies between 10 and 100 mm, although it is possible to have e.g. 5 mm or even 20 cm.

The drill itself was a brushless motor having a torque of 6 nm and a maximum revolution of 1100 rpm. The use of the brushless motor allows the proper calculation of the power based on U and 1. There is no loss due to variation of the electrical resistance of the motor.

According to a preferred embodiment of the invention the energy is directly calculated based on the electric power consumption together with the geometrical values of the drill.

Additionally, it is possible to measure the torque, the piercing speed and the rotational speed of the drill bit, although this is not a mandatory feature of such an embodiment. The use of these parameters allow also the determination of the specific energy (per volume, when used in connection with the geometrical values of the drill).

Since the important parameter is the energy used to drill the hole or to cut and remove the bone, the time factor of drilling or sawing is in principle irrelevant. However, since every drilling action also comprises some kind of friction, it is preferred in order to enhance the quality of the result to work in the usual time frame for drilling such holes or to cut the bone.

The usual applications where it is quite crucial to know more about the bone rely to femoral head replacements, although these interventions can be related to surgical interventions on any portion of the musculoskeletal system. Besides drilling it is also possible to take into account the movement back and forth of a saw, e.g. when an artificial knee implant is to be affixed.

Beside using electrical signals of the drill or saw 100 it is also optionally possible to use the power of an air operated machining tool 105, which is given by the following:

$$P = \frac{1}{2}\dot{m}(v_1{}^2 - v_2{}^2)$$

$P$ power

$\dot{m}$ mass flow

$v_1$ air velocity 1

$v_2$ air velocity 2

The integration into the given procedure is analog to an electric powered system using the mass flow or the air velocities as mentioned in box 125. The terms related to the power are simply exchanged by corresponding air powered device values.

LIST OF REFERENCE SIGNS

| | | | |
|---|---|---|---|
| 10 | drill / surgical instrument | 135 | drill bit parameters |
| 11 | drill bit | 140 | x-ray (or DEXA) exposure result |
| 12 | connection | | |
| 18 | current consumption | 145 | bone owner related data |
| 19 | revolution per time unit | 150 | calculus unit |
| 20 | control unit | 160 | screen representation |

(continued)

| | | | |
|---|---|---|---|
| 30 | second sensor unit, C-arm, x-ray detector | 170 | threshold comparison |
| | | 200 | power (y-axis) |
| 32 | connection | 201 | idle power consumption |
| 40 | x-ray source | 210 | time (x-axis) |
| 41 | x-ray beam | 211 | start time |
| 50 | bone | 212 | stop time |
| 51 | local region of bone | 213 | stop of drill system |
| 52 | drilled hole | 220 | curve related to one drill event |
| 100 | drill system | | |
| 105 | air operated drill system | 225 | integral over time |
| 110 | volume sensor unit / depth sensor | 230 | curve related to a further drill event |
| 115 | drill bit parameters (electric) | 235 | integral over time |
| 120 | drill further parameters | 240 | measured power consumption |
| 125 | drill power parameters (air operated) | 245 | measured integral over time |
| 130 | volume parameters | | |

**Claims**

1. A surgical machining instrument (10), especially a surgical drilling or sawing instrument, for determining the local material properties, especially the mechanical resistance of a bone (50), in connection with the machining action, comprising a first sensor unit adapted to determine energy expenditure during the machining action and a control unit (20) adapted to convert (150) the measured data in a value (160) of the local material properties, **characterized in that** the instrument comprises a second sensor unit adapted to determine the volume of removed material during the machining action and being time synchronized with the first sensor unit and **in that** the control unit (20) is adapted to determine the applied specific energy as energy per removed material, especially bone, volume (225, 235, 245) over time based on the data of the sensor units and to convert (150) the measured data in a value (160) for the mechanical resistance of said bone.

2. The surgical instrument according to claim 1, wherein the control unit (20) is adapted to take into account as parameter the kind of machining tool (105, 110, 115, 120), especially if it is a drilling tool or a saw, especially the diameter of the drill or the thickness of the saw blade, respectively.

3. The surgical instrument according to claim 1 or 2, wherein the second sensor unit (110) comprises a depth gauge adapted to monitor, during the machining, the advance of the machining instrument into the material to determine the volume of removed material.

4. The surgical instrument according to claim 1 or 2, wherein the second sensor unit (30, 40) comprises an x-ray apparatus and an x-ray detector adapted to monitor, during the machining, the advance of the machining instrument into the material to determine the volume of removed material.

5. A system comprising a surgical machining instrument (10) according to any one of claims 1 to 4 and a dual energy x-ray apparatus (40) for determining the local mechanical resistance of a bone (50) in connection with the machining action, **characterized in that** the control unit (20) is adapted to receive measured dual energy x-ray beam data (41) from an x-ray detector (30) relating to the local bone portion (50) to be machined and is further adapted to calculate the calcium content and calcium based stability of the bone (50) obtained through said dual energy x-ray absorptiometry measurement and to calculate the collagen based stability of the bone (50) taking into account the overall stability of the bone (50) calculated based on said measured energy consumption data related to the local mechanical resistance of said bone (50).

6. The system according to claim 5, wherein the control unit (20) adapted to convert (150) the measured data in a value (160) of the local material properties converts said data real-time based on said mathematical correlation (150).

7. The surgical instrument according to any one of claims 1 to 6, wherein the machining instrument is electric driven and wherein the first sensor unit comprises sensors detecting current consumption (18) and applied voltage over time.

8. The surgical instrument according to claim 1 to 6, wherein the machining instrument is air driven and wherein the first sensor unit comprises sensors detecting the mass flow or the air velocities of an air driven instrument.

9. A method for determining the local mechanical resistance of a bone being machined in situ or ex situ, comprising the steps of determining values relative to the energy consumption for the local bone portion being machined by a surgical machining instrument (10) according to any one of claims 1 to 8, especially a surgical drilling or sawing instrument, detected by a first sensor unit, determining the volume of removed material during the machining action detected by a second sensor unit (110) and calculating, during the machining action, the applied specific energy as energy per volume (225, 235, 245) based on the energy consumption and removed volume material over time, and converting (150) the measured data in a value (160) of the local mechanical resistance of said bone.

10. The method according to claim 9, wherein the energy consumption is determined based on current consumption of an electric driven machining instrument and the applied voltage is measured over time and integrated into said energy per volume value.

11. The method according to claim 9 or 10, wherein the method further comprises the step of determining a calcium content and calcium based stability value for the local bone through x-raydual energy x-ray absorptiometry by detection of x-ray beams transmitted through the bone portion in question before machining, wherein a collagen stability value for the local bone is subsequently calculated through deduction from said overall local mechanical resistance of said bone.

Fig. 1

Fig. 2

**Fig. 3**

Fig. 4

Fig. 5

Europäisches
Patentamt

European
Patent Office

Office européen
des brevets

## EUROPEAN SEARCH REPORT

Application Number

EP 13 00 3805

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 00/15138 A1 (NOBEL BIOCARE AB [SE]; DAWOOD ANDREW [GB]; PETERSSON ANDERS [SE]) 23 March 2000 (2000-03-23) * page 5, last paragraph; figures 1,6 * * abstract * * page 11, paragraph 2 * | 1-9 | INV. A61B5/00 A61B17/16 |
| X | US 2011/245833 A1 (ANDERSON WAYNE [US]) 6 October 2011 (2011-10-06) * paragraphs [0004] - [0010], [0073], [0074], [0088] - [0091], [0102] * | 1-9 | |
| L | Florian Schellenberg ET AL: "Curriculume Vitae General Information MSc ETH in Human Movement Sciences with a Major in Biomechanics", , 10 March 2014 (2014-03-10), XP055116580, Retrieved from the Internet: URL:http://n.ethz.ch/~scfloria/Lebenslauf.pdf [retrieved on 2014-05-06] * page 4, item 7 * | | |

TECHNICAL FIELDS
SEARCHED (IPC)

A61B

The present search report has been drawn up for all claims

| Place of search | Date of completion of the search | Examiner |
|---|---|---|
| Berlin | 23 June 2014 | Clevorn, Jens |

EPO FORM 1503 03.82 (P04C01)

**ANNEX TO THE EUROPEAN SEARCH REPORT
ON EUROPEAN PATENT APPLICATION NO.**                    EP 13 00 3805

This annex lists the patent family members relating to the patent documents cited in the above-mentioned European search report.
The members are as contained in the European Patent Office EDP file on
The European Patent Office is in no way liable for these particulars which are merely given for the purpose of information.

23-06-2014

| Patent document cited in search report | | Publication date | Patent family member(s) | | Publication date |
|---|---|---|---|---|---|
| WO 0015138 | A1 | 23-03-2000 | AU | 6376199 A | 03-04-2000 |
| | | | CA | 2339660 A1 | 23-03-2000 |
| | | | EP | 1113762 A1 | 11-07-2001 |
| | | | JP | 2002524195 A | 06-08-2002 |
| | | | SE | 9803153 A | 18-03-2000 |
| | | | US | 6547565 B1 | 15-04-2003 |
| | | | WO | 0015138 A1 | 23-03-2000 |
| US 2011245833 | A1 | 06-10-2011 | US | 2011245833 A1 | 06-10-2011 |
| | | | WO | 2011123703 A1 | 06-10-2011 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 102009033364 A1
- US 6591698 B
- WO 00115138 A1
- US 20080300510 A1
- US 2011245833 A